# EUROPÄISCHE PATENTSCHRIFT

(11) **EP 0 846 186 B1**
(45) Veröffentlichungstag und Bekanntmachung des Hinweises auf die Patenterteilung: **20.02.2008**
(21) Anmeldenummer: 96929253.1
(22) Anmeldetag: 13.08.1996
(51) Int. Cl.: C12Q 1/68, G01N 33/50

(54) **EXTRAKTION, AMPLIFIKATION UND SEQUENTIELLE HYBRIDISIERUNG VON PILZZELLEN-DNA SOWIE VERFAHREN ZUM NACHWEISEN VON PILZZELLEN IN KLINISCHEM MATERIAL**
EXTRACTION, AMPLIFICATION AND SEQUENTIAL HYBRIDISATION OF FUNGUS CELL DNA AND A PROCESS FOR DETECTING FUNGUS CELLS IN CLINICAL MATERIAL
EXTRACTION, AMPLIFICATION ET HYBRIDATION SEQUENTIELLE D'ADN DE CELLULES DE CHAMPIGNONS ET PROCEDE DE DETECTION DE CELLULES DE CHAMPIGNONS DANS UN MATERIAU CLINIQUE

(30) Priorität: 17.08.1995 DE 19530332; 17.08.1995 DE 19530333; 17.08.1995 DE 19530336
(43) Veröffentlichungstag der Anmeldung: 10.06.1998
(60) Teilanmeldung: 98103296.4
(73) Patentinhaber: Myconostica Limited, Sharston Road Sharston Manchester M22 4SN (GB)
(72) Erfinder: EINSELE, Hermann, D-72076 Tübingen (DE)
(74) Vertreter: Otten, Hajo
(86) Internationale Anmeldenummer: PCT/EP1996/003580
(87) Internationale Veröffentlichungsnummer: WO 1997/007238

(56) Entgegenhaltungen:
- EP-A- 0 285 439
- EP-A- 0 335 633
- EP-A- 0 422 869
- EP-A- 0 574 267
- WO-A-92/08807
- WO-A-93/23568
- JP-A- 8 089 254
- US-A- 4 130 395
- US-A- 5 426 026
- US-A- 5 426 027
- US-A- 5 434 048
- JOURNAL OF CLINICAL MICROBIOLOGY, Bd. 32, Nr. 1, Januar 1994, WASHINGTON, US, Seiten 228-231, XP000645486 HOLMES ET AL.: "Detection of C. albicans and other yeasts in blood by PCR"
- BONE MARROW TRANSPLANTATION, 13.März 1994, Seite 184 XP000645489 EINSELE H ET AL: "DETECTION OF VARIOUS FUNGAL PATHOGENS IN BLOOD SAMPLES BY PCR"

## Beschreibung

Die vorliegende Erfindung betrifft allgemein Nukleotidsequenzen sowie ein Verfahren zum Nachweisen von Pilzzellen in klinischem Material, in das die Nukleotidsequenzen eingesetzt werden können.

Derartige Nukleotidsequenzen und Verfahren sind aus der EP 0 422 869 A2 bekannt.

Derartige Verfahren sind außerdem aus der Praxis bekannt, sie beruhen standardmäßig auf einer Anzüchtung von Pilzspezies aus klinischem Material auf entsprechenden Nährmedien.

Durch diese Anzüchtung z.B. in Petrischalen und den Nachweis anhand der gewachsenen oder auch nicht gewachsenen Kolonien sind die Nachweisgeschwindigkeit sowie -empfindlichkeit insbesondere wegen des langsamen Wachstumes der Pilzspezies stark beeinträchtigt. Die Diagnose der invasiven Pilzinfektion wird daher häufig erst durch eine äußerst komplikationsträchtige Biopsie eines Organes oder sogar erst nach dem Tod des Patienten gestellt.

Das Interesse an Verfahren zum Nachweisen von Pilzzellen ist vor dem Hintergrund zu sehen, daß insbesondere in den letzten Jahren Pilzspezies als bedeutende nosokomiale Pathogene eine erhebliche Bedeutung für immunsupprimierte Patienten erlangt haben. Vor allem nach Knochenmarkstransplantationen (KMT), aber auch nach Leber-, Nieren-, Pankreas-, Herz- und Herz-Lungen-Transplantationen haben invasive Pilzinfektionen erheblich zugenommen. So ist es z.B. im Jahr 1994 vor allem in französischen KMT-Zentren zu einer solchen Häufung vor allem von Aspergillusinfektionen gekommen, daß diese Zentren mehrere Monate geschlossen werden mußten.

Neben den organtransplantierten Patienten sind aber auch Patienten mit Krebserkrankung, vor allem nach Chemotherapie oder chirurgischen Eingriffen, Verbrennungspatienten sowie Patienten auf chirurgischen und neonatalen Intensivstationen zunehmend von invasiven Pilzinfektionen betroffen. Sobald es bei diesen Patientenkollektiven zu einer Beteiligung eines Organsystemes oder gar mehrerer Organsysteme kommt, beträgt die Sterblichkeit an dieser infektiösen Komplikation zwischen 80 und 100 %.

Nur durch eine frühzeitige Diagnose kann hier der Behandlungserfolg verbessert werden. Wegen der mit den eingangs erwähnten Standardnachweisverfahren verbundenen Nachteile werden intensive Bemühungen unternommen, eine frühzeitige Sicherung der Diagnose einer systemischen Pilzinfektion zu ermöglichen.

In der eingangs genannten EP 0 422 869 A2 werden Nukleotidsequenzen sowie Verfahren zum Nachweis pathogener Candida-Hefen beschrieben. Dort werden Hybridisierungssonden beschrieben, die mit Bereichen aus dem 18S rRNA Gen von Candida-spezies hybridisieren. Die Hybridisierung wird in "Dot Blot"-Verfahren oder in sogenannten "Sandwich"-Hybridisierungsassays getestet. Es werden außerdem zwei Primer beschrieben, mit denen fast das gesamte 18S rRNA Gen von Candida-Hefen in einer PCR-Reaktion amplifiziert werden kann.

Aus der Veröffentlichung "Detection of various fungal pathogenes in blood samples" in: EBMT 1995, Vol. 15, Suppl. 2, März 1995, Abstract Book, Abstract 432, S. 103, ist es bekannt, ein DNA-Segment eines Pilz-Genes mittels des PCR-Verfahrens zu amplifizieren, um eine Vielzahl von Pilz-Pathogenen im Blut zu identifizieren. Durch zusätzliche Hybridisierung mit speziesspezifischen Oligonukleotiden konnten ferner verschiedene Pilzspezies voneinander unterschieden werden.

Die wesentlichen Probleme des Nachweises von Pilzinfektionen mit Hilfe molekularbiologischer Techniken sind dabei auf die sehr komplexe Zusammensetzung der Pilzzellenwand zurückzuführen, die bisher zeitaufwendige aber auch teure Extraktionsverfahren erforderlich machen.

Ein weiteres Problem besteht darin, daß eine zunehmende Zahl von Pilzspezies bei den immunsupprimierten Patienten bedrohliche Infektionen auslösen kann, woraus die Notwendigkeit resultiert, eine Fülle von verschiedenen Pilzgattungen und -stämmen bei diesen Patienten erfassen und bestimmen zu müssen. Da sich die Therapie der Infektionen bei verschiedenen Pilzspezies nämlich unterscheidet, ist es folglich erforderlich, nicht nur alle Pilzspezies erfassen, sondern diese auch differenzieren und identifizieren zu können.

Vor diesem Hintergrund ist es Aufgabe der vorliegenden Erfindung, Nukleotidsequenzen sowie ein Verfahren bereitzustellen, mit dem eine frühzeitige Diagnose einer Pilzinfektion möglich wird.

Das Verfahren soll dabei möglichst schnell und einfach durchzuführen sein, möglichst viele Pilzspezies erfassen und in einer Weiterbildung auch identifizieren können.

Diese Aufgabe wird durch die Nukleotidsequenzen SEQ ID-No: 1 und SEQ ID-No: 2 aus dem beiliegenden Sequenzprotokoll gelöst.

Diese Nukleotidsequenzen sind bevorzugt als Primer zur Amplifikation eines Abschnitts aus dem Pilzgen für die 18ssu rRNA von einer Vielzahl von Pilzspezies in einer Polymerase-Kettenreaktion zu verwenden.

Der Erfinder der vorliegenden Anmeldung hat nämlich überraschenderweise gefunden, daß diese beiden Nukleotidsequenzen als Primer für die verschiedensten Pilzspezies verwendbar sind, die im Klinikalltag relevant sind. Durch Verwendung dieser beiden Primer in der PCR-Reaktion werden Verstärkungsprodukte erzeugt, die eine Länge von ca. 500 Basenpaaren haben und somit auch leicht weiterzuverarbeiten sind, da sie z.B. leicht über Gelelektrophorese nachgewiesen werden können. Auf diese Weise ergibt sich also ein identisches Nachweisverfahren für alle pathogenen Pilzspezies, da gefunden wurde, daß die beiden Primersequenzen an die verschiedene Pilz-DNA binden.

Die der Erfindung zugrunde liegende Aufgabe wird auch durch ein Verfahren zum Nachweisen von Pilz-DNA in klinischem Material unter Extrahieren von Pilz-DNA aus Vollblut gelöst, das die folgenden Schritte aufweist:
a) Isolation überwiegend intakter Pilzzellen aus dem Vollblut, durch
   a1) Aufschließen der im Vollblut befindlichen Blutzellen;
   a2) Isolation überwiegend intakter Pilzzellen von zellulärer DNA;
b) Extrahieren von DNA aus den isolierten Pilzzellen durch b1) Aufschließen der isolierten Pilzzellen, und
   b2) Isolation der Pilz-DNA;
c) Amplifikation zumindest eines Segments der nachzuweisenden Pilz-DNA durch Polymerase-Kettenreaktion mit den Nukleotidsequenzen SEQ ID-No: 1 und SEQ ID-No: 2 aus dem beiliegenden Sequenzprotokoll als Primer, und
d) gegebenenfalls Nachweis der Amplifikationsprodukte.

Dadurch wird ein Verfahren geschaffen, das bei hoher Sensitivität über den Nachweis von pilzspezifischer DNA den sehr frühen Nachweis von Pilzinfektionen ermöglicht.

Das Verfahren der Extraktion von Pilz-DNA ist dabei geeignet, insbesondere in der Diagnostik auch bei sehr geringen Mengen von Pilzen in dem vorliegenden Vollblut noch zuverlässig Pilz-DNA extrahieren zu können. Ferner kann dieses Verfahren schnell und so einfach durchgeführt werden, daß es auch im Klinikalltag und ggf. von angelerntem Personal angewendet werden kann.

Durch das erfindungsgemäße Verfahren wird eine sehr schnelle und sichere Trennung der Pilz-DNA von zellulärer DNA erreicht. Durch den Aufschluß der im Vollblut befindlichen Blutzellen wird nämlich in dieser Ausgangslösung die zelluläre DNA freigesetzt, woraufhin dann die durch das bisherige Aufschlußverfahren noch nicht oder zumindest noch nicht vollständig aufgeschlossenen Pilzzellen durch schnelle und einfache Verfahren, wie z. B. eine Zentrifugation von der freien zellulären DNA getrennt werden können. Bei dem daraufhin folgenden Aufschließen der so isolierten Pilzzellen kann mit großer Sicherheit davon ausgegangen werden, daß sich keine oder nur noch unerhebliche Mengen an zellulärer DNA in der Lösung befindet. Das im Schritt a1) durchzuführende Aufschließen der Blutzellen muß folglich so erfolgen, daß die Pilzzellen noch nicht lysiert werden. Da die Pilzzellwand deutlich komplexer ist als die Zellwand von Blutzellen, kann dies durch geeignete vorsichtige Aufschlußverfahren sichergestellt werden.

Der Verfahrensschritt a1) hat jedoch noch einen weiteren Vorteil, durch ihn werden nämlich ggf. phagozytierte Pilzzellen wieder freigesetzt, so daß insbesondere für die Diagnostik auch noch sehr geringe Mengen von Pilzen in dem vorliegenden Vollblut extrahiert werden können. Bei dem neuen Verfahren ist es nämlich nicht mehr erforderlich, daß zumindest einige Pilzzellen in dem Vollblut frei in Lösung sind, es reicht völlig aus, wenn einige wenige Pilzzellen nach Phagozytose z.b. in Granulozyten oder Makrophagen vorliegen.

Dabei ist es insbesondere bevorzugt, wenn im Schritt a) folgende Schritte durchgeführt werden:
a1.1) Lysis der roten Blutkörperchen durch osmotische Hämolyse;
a1.2) enzymatisches Aufschließen der weißen Blutkörperchen; und
a2.1) Zentrifugation des so behandelten Vollblutes und Verwendung des Pellet in den nächsten Verfahrensschritten.

Hier ist von Vorteil, daß in den Schritten a1.1) und a1.2) zwar zuverlässig die Blutzellen aufgeschlossen werden, die Pilzzellen selbst jedoch noch nicht beschädigt sind. Durch die folgende Zentrifugation ist dann eine sehr sichere Trennung zwischen der inzwischen freigesetzten zellulären DNA der Blutzellen und Zellbruchstücken der Blutzellen einerseits sowie noch überwiegend intakten Pilzzellen andererseits möglich. Auf diese Weise wird außerdem sichergestellt, daß keine Pilz-DNA verlorengeht, da diese noch in den im Pellet zu findenden Pilzzellen vorhanden ist. Zusammengefaßt liegen die Vorteile der obigen Schritte also darin, daß zum einen auch geringste Konzentrationen von Pilzzellen erfaßt werden und daß zum anderen die isolierte Pilz-DNA allenfalls gering mit zellulärer DNA verunreinigt ist, so daß eine hohe Sensitivität und Spezifität bei den nachfolgenden diagnostischen Schritten möglich ist, da das Verhältnis von Pilz- zu zellulärer DNA zugunsten der Pilz-DNA deutlich verbessert wurde.

In einer Weiterbildung ist es dann bevorzugt, wenn im Schritt b) folgende Schritte durchgeführt werden:
b1.1) alkalisches Lysieren und enzymatische Behandlung der Pilzzellen.

Es hat sich herausgestellt, daß durch diese einfachen Verfahrensschritte ein sicheres Aufschließen der Pilzzellen möglich ist, die aus dem Pellet des Verfahrensschrittes a2.1) wieder aufgenommen wurden.

Weiterhin ist es bevorzugt, wenn im Schritt a1.1) die Lysis der roten Blutkörperchen mit Hilfe einer hypotonen Lösung erfolgt, vorzugsweise mit einer Endkonzentration von ca. 10 mM Tris pH 7, 6, 5 mM MgCl₂ und 10 mM NaCl, und wenn im Schritt a1.2) das enzymatische Aufschließen der weißen Blutkörperchen in einer Lösung mit einer Endkonzentration von 200 µg/ml Proteinase K, 10 mM Tris pH 7,6, 10 mM EDTA pH 8, 0, 50 mM NaCl und 0,2 % SDS erfolgt.

In einer Weiterbildung wird die Lösung aus Schritt a1.2) für 100-140 min, vorzugsweise 120 min bei 60-70°C, vorzugsweise bei 65°C inkubiert.

Es wurde gefunden, daß auf diese Weise die Blutzellen zuverlässig und vollständig aufgeschlossen werden können, wobei eine Beschädigung der Pilzzellen vermieden wird, so daß nach diesen Verfahrensschritten sowohl die in dem Ausgangsblut frei in Lösung befindlichen als auch die phagozytierten Pilzzellen relativ unbeschädigt vorliegen und abzentrifugiert werden können, ohne ihre DNA dabei zu verlieren.

Ferner ist es bevorzugt, wenn der Schritt b1.1) die folgenden Schritte umfaßt:
- Inkubieren der im Pellet aus Schritt a2.1) befindlichen Pilzzellen bei 90-98°C, vorzugsweise 95°C für 5-15 min, vorzugsweise 10 min, in einer Lösung mit 50 mM NaOH,
- Neutralisation mit 1 M Tris-HCl pH 7,0,
- Enzymatische Behandlung mit Zymolyase für 50-70 min, vorzugsweise 60 min bei 30-40°C, vorzugsweise 37°C,
- Proteindenaturierung durch Inkubation mit Tris/EDTA bei 60-70°C, vorzugsweise 65°C, für 10-30 min, vorzugsweise 20 min.

Es wurde gefunden, daß durch diese Verfahrensschritte ein sicheres Aufschließen der Pilzzellen mit daraufhin erfolgender vollständiger Freigabe der Pilz-DNA möglich ist, so daß die Pilz-DNA nunmehr frei in Lösung ist und von den Zellbruchstücken der Pilzzellen isoliert werden kann.

Dabei ist es bevorzugt, wenn in diesem Zusammenhang der Schritt b2) folgende Schritte umfaßt:,
- Proteinpräzipitation mit 5 M Kaliumazetat, und
- DNA-Präzipitation des Überstandes in eiskaltem Isopropanol.

Diese Verfahrensschritte sind sehr einfach durchzuführen, zunächst wird durch Zugabe von Kaliumazetat das Protein herausgefällt, dann der Überstand abgenommen und mit eiskaltem Isopropanol versetzt, so daß die DNA ausfällt. Diese ausgefallene DNA kann dann für die weiteren Verfahrensschritte herangezogen werden, also zum Nachweis und zur Identifizierung einer Pilzinfektion dienen.

Bei dem insoweit beschriebenen Verfahren ist also zusammengefaßt als erstes von Vorteil, daß die Pilzspezies sozusagen an Hand ihrer DNA allgemein nachgewiesen und dann speziell identifiziert werden kann. Die Pilz-DNA wird dabei nicht direkt aus dem Vollblut extrahiert, sondern zunächst erfolgt eine Trennung der Pilzzellen von zellulärer DNA, um die Sensitivität und Spezifität des Nachweises zu erhöhen. Mit anderen Worten, wenn nur sehr wenige Pilzzellen in dem Vollblut vorhanden sind, so könnte die daraus extrahierte sehr geringe Pilz-DNA-Menge ggf. vor dem Hintergrund der in sehr hoher Konzentration vorliegenden zellulären DNA nicht nachgewiesen werden, so daß die erwähnte Trennung hier große Vorteile bezüglich der Sensitivität bringt.

Ein weiterer Vorteil bei dem neuen Verfahren liegt darin, daß auch phagozytierte Pilzzellen für den Nachweis zur Verfügung stehen, da zunächst die Blutzellen des Vollblutes aufgeschlossen werden, ohne daß dabei die Pilzzellen, seien sie frei in Lösung oder in phagozytiertem Zustand, beschädigt werden. Erst nach Abtrennung der Pilzzellen von der zellulären DNA und den verbleibenden Zelltrümmern der Blutzellen werden dann die Pilzzellen aufgeschlossen. Das hierzu verwendete Verfahren ist in der Lage, trotz der sehr komplexen Pilzzellwände für einen vollständigen Aufschluß zu sorgen, ohne daß dabei die Pilz-DNA zerstört wird.

Gegenstand der vorliegenden Anmeldung ist ebenfalls ein Kit zur Durchführung des oben beschriebenen Verfahrens. Ein derartiges Kit kann eine Zusammenstellung sämtlicher Stammlösungen enthalten, wie sie für die genannten Verfahrensschritte im einzelnen benötigt werden. Es ist jedoch auch möglich, in diesem Kit nur die nicht üblicherweise in einem Labor vorrätigen Stammlösungen vorzusehen, so daß bspw. auf die Tris-Puffer etc. verzichtet werden kann, aber zumindest die Proteinase K und die Zymolyase in dem Kit vorhanden sind.

Mit den Schritten c) und d) erfolgt der Nachweis der extrahierten Pilz-DNA. Die nachzuweisende Pilz-DNA wird dabei vorteilhafterweise so extrahiert bzw. isoliert, wie es oben beschrieben ist. Es ist jedoch auch möglich, die Pilz-DNA z. B. durch geeignete Dichtegradienten-Zentrifugation, spezifische Fällungsschritte mit DNA-Sonden etc. zu gewinnen.

Die Pilz-DNA wird zwar vorteilhaft zum Zwecke der Diagnostik von Pilzinfektionen herangezogen, sie kann jedoch auch anderweitig weiterverarbeitet werden. Die Pilz-DNA kann dafür über Agarplatten oder aus dem Blut von Tieren gewonnen werden, die speziell mit den interessierenden Pilzspezies infiziert wurden. Aus der so gewonnenen Pilz-DNA können z. B. DNA-Sonden herausgeschnitten werden, die für Nachweisreaktionen verwendet oder in Plasmide eingebaut werden können. Es sind dabei Anwendungen im ganzen Bereich der Grundlagenforschung, Diagnostik, Therapeutik, industriellen Gentechnologie etc. denkbar.

Durch den neuen Nachweisschritt werden dabei in einem einzigen Verfahren viele verschiedene Pilzspezies auch bei sehr geringer Konzentration in der Ausgangslösung erfaßt, so daß z. B. im Rahmen der Diagnostik sehr schnell und sehr frühzeitig eine Aussage möglich ist, ob überhaupt eine Pilzinfektion vorliegt. Das neue Verfahren ist darüber hinaus schnell und so einfach durchzuführen, daß es auch im Klinikalltag angewendet und ggf. von angelerntem Personal durchgeführt werden kann.

Die Amplifikation erfolgt dabei mittels PCR (Polymerase-Kettenreaktion), wobei der Nachweis über Gelelektrophorese, optische Dichte, Anfärben mit spezifisch an Nukleinsäure bindenden Markern etc. erfolgen kann. Diese Verfahren sind hochspezifisch und sehr sensitiv, so daß sie die gewünschten Eigenschaften für Diagnostik, Therapeutik, industrielle Gentechnologie etc. mit sich bringen.

Die PCR erfolgt unter Verwendung der zwei Primer mit den Nukleotidsequenzen SEQ ID-No: 1 und SEQ ID-No: 2 aus dem beiliegenden Sequenzprotokoll, die an die DNA von einer Vielzahl von verschiedenen Pilzspezies binden, und zwar an das Pilz-Gen für die 18 ssu-rRNA.

Hier ist von Vorteil, daß auf einfache und inzwischen etablierte Weise DNA in ausreichender Menge erzeugt werden kann, die dann leicht nachzuweisen, aber auch weiterverwendet werden kann, um die betreffenden Pilzspezies zu identifizieren.

Dabei ist es bevorzugt, wenn die Polymerase-Kettenreaktion mit folgendem Zyklus durchgeführt wird:
c1) Denaturieren für 0,3-1 min, vorzugsweise für 0,5 min bei 90-96°C, vorzugsweise bei 94°C;
c2) Hybridisieren für 0,5-1,5 min, vorzugsweise für 1,0 min bei 58-64°C, vorzugsweise bei 62°C, und
c3) Extension für 1,5-2,5 min, vorzugsweise für 2,0 min bei 68-75°C, vorzugsweise bei 72°C.

Dabei ist es bevorzugt, wenn vor Beginn der Zyklusschritte ein Denaturierungsschritt von 5-9 min, vorzugsweise von 3 min bei 90-96°C, vorzugsweise bei 94°C erfolgt.

Es wurde gefunden, daß mit den obigen Bedingungen die PCR-Reaktion sehr reproduzierbar und vor allem hochspezifisch abläuft, so daß sichergestellt ist, daß die Amplifikationsprodukte auch tatsächlich Segmente der ursprünglichen Pilz-DNA sind.

Weiter ist es bevorzugt, wenn im Schritt d) die Amplifikationsprodukte gelelektrophoretisch, vorzugsweise auf einem Agarosegel, nachgewiesen werden und dazu vorzugsweise mit Ethidiumbromid angefärbt werden.

Hier ist von Vorteil, daß ein bekanntes und gut etabliertes Nachweisverfahren verwendet wird, um aufzeigen zu können, daß bei der PCR-Reaktion tatsächlich Amplifikationsprodukte entstanden sind, die auf eine Pilzinfektion hinweisen.

Weiter ist es dann bevorzugt, wenn eine DNA-Sequenz aus dem Pilz-Gen für die 18ssu-rRNA amplifiziert wird.

Der Erfinder der vorliegenden Anmeldung hat nämlich erkannt, daß dieses Pilz-Gen bei den verschiedenen Pilz-Stämmen und -Gattungen ein derartiges Sequenzsegment aufweist, das einerseits von zwei Bindungsregionen für Primer flankiert wird, die für alle Pilz-Stämme und -Gattungen identisch sind, daß aber andererseits die Sequenz dieses Segmentes für die verschiedenen Pilz-Stämme und -Gattungen so verschieden ist, daß sie zum Nachweis der einzelnen Pilzspezies und -Gattungen verwenden werden kann.

Die Erfindung betrifft ferner einen Kit zum Nachweisen von Pilz-DNA in einer Untersuchungslösung, wobei dieser Kit an die DNA von einer Vielzahl von Pilzspezies bindende Primer für eine Polymerase-Kettenreaktion enthält. Vorzugsweise enthält dieser Kit als Primer die Nukleotidsequenzen SEQ ID-No: 1 und SEQ ID-No: 2 aus dem Sequenzprotokoll.

Die Erfindung betrifft ferner einen Kit zur Durchführung des oben erwähnten Verfahrens.

Bei einem derartigen Kit ist von Vorteil, daß sämtliche erforderlichen Lösungen und insbesondere die erforderlichen Primer zusammengestellt werden, so daß sie für Routineverfahren verwendet werden können. So ist es zum Beispiel im Laboralltag möglich, auf diese neuen Kits zurückzugreifen, um in vorliegenden Untersuchungslösungen Pilzspezies überhaupt nachweisen zu können. Ferner kann dieser Kit dazu verwendet werden, bestimmte Segmente der nachgewiesenen Pilzspezies hochzuverstärken, die dann bspw. für weitere Identifizierungsverfahren verwendet werden können.

Gemäß der vorliegenden Anmeldung ist auch isoliert oder zusammen mit den oben beschriebenen Verfahrensabschnitten 1 und 2 der dritte Verfahrensabschnitt, also die Bestimmung der Pilzspezies anhand der extrahierten und bereits nachgewiesenen Pilz-DNA.

Die Bestimmung der Pilzspezies soll dabei insbesondere für die Diagnostik schnell und so einfach durchzuführen sein, daß sie auch im Klinikalltag und ggf. von angelerntem Personal durchgeführt werden kann.

Diese Aufgabe wird erfindungsgemäß dadurch gelöst, daß folgender zusätzlicher Schritt durchgeführt wird:
e) Bestimmen von für die Pilzspezies kennzeichnenden Nucleotidsequenzabschnitten der in der Untersuchungslösung enthaltenen DNA.

Da das Nachweisverfahren auf DNA-Ebene durchgeführt wird, ist es völlig ausreichend, wenn bestimmte Segmente der Pilz-DNA bestimmt werden, sofern diese Segmente für die Pilzspezies jeweils spezifisch ist. Dabei kann auf Standardverfahren zurückgegriffen werden, indem zumindest teilweise die Amplifikationsprodukte sequenziert oder ansequenziert werden, das Schmelzverhalten der Doppelstränge untersucht wird, etc.

Diese Verfahren sind in der Regel so schnelle und einfach durchzuführen, daß sie auch von angelerntem Personal vorgenommen werden können.

Dabei ist es bevorzugt, wenn im Schritt e) die Pilz-DNA oder Segmente der Pilz-DNA mit DNA-Sonden hybridisiert werden, die für bestimmte Pilzstämme und/oder -spezies spezifisch sind, wobei der Test der erfolgten Hybridisierung damit zur Identifizierung der Pilzspezies führt.

Hier ist von Vorteil, daß ein sehr schnell und einfach durchzuführendes Hybridisierungsverfahren zum Nachweis verwendet wird. Hier werden spezifische Sonden eingesetzt, die für die jeweiligen Pilzspezies spezifisch sind, so daß sie ausschließlich an die amplifizierten Segmente dieser Pilzspezies binden. Derartige Verfahren lassen sich z.B. auf Gelen durchführen, wobei die Hybride dann durch Anfärben kenntlich gemacht werden. Ein weiteres Verfahren besteht darin, die optisch unterschiedlichen Verhaltensweisen von Einzelsträngen und doppelsträngigen Regionen, z.B. in der optischen Dichte, im Dichroismus oder ähnlichem auszunutzen.

Weiter ist es bevorzugt, wenn zum Testen der erfolgten Hybridisierung die Sonden mit Digoxigenin markiert und die Hybride z.B. nach der Southern Blot-Methode nachgewiesen werden.

Durch diesen Schritt vereinfacht sich das Verfahren noch einmal, die Sonden selbst sind bereits mit dem entsprechenden Marker versehen, der mit einem bekannten Verfahren nach der Hybridisierung durch eine vorteilhafterweise sogar visuell zu erkennende Farbstoffreaktion die Hybridbildung anzeigt.

Dabei ist es bevorzugt, wenn als DNA-Sonden eine oder mehrere der Nucleotidsequenzen SEQ ID-No: 3 bis SEQ ID-No: 8 aus dem beiliegenden Sequenzprotokoll verwendet werden, wobei die DNA-Sonden vorzugsweise sequentiell nacheinander in der Reihenfolge SEQ ID-No: 3, SEQ ID-No: 8, SEQ ID-No: 6, SEQ ID-No: 7, SEQ ID-No: 4 und SEQ ID-No: 5 eingesetzt werden und jeweils die Hybridisierung getestet wird.

Durch dieses sequentielle Hybridisieren können die einzelnen Pilzspezies in der Reihenfolge ihrer Häufigkeit getestet werden, so daß sich das Nachweisverfahren deutlich beschleunigen läßt.

Die Erfindung betrifft ferner eine der Nucleotidsequenzen SEQ ID-No: 3 - SEQ ID-No: 8 aus dem beiliegenden Sequenzprotokoll.

Die Erfindung betrifft ferner eine Verwendung einer oder mehrerer dieser Nucleotidsequenzen als DNA-Sonde zum Identifizieren von Pilzspezies.

Hier ist von Vorteil, daß durch die 6 angegebenen Nucleotidsequenzen spezifisch sämtliche klinisch interessierenden Pilzspezies voneinander unterschieden werden können.

Die Nucleotidsequenz SEQ ID-No: 3 dient dabei zum Nachweis der Pilzspezies Candida albicans, SEQ ID-No: 4 zum Nachweis von Candida glabrata, SEQ ID-No: 5 zum Nachweis von Candida krusei, SEQ ID-No: 6 zum Nachweis von Candida tropicalis, SEQ ID-No: 7 zum Nachweis von Candida parapsilosis und SEQ ID-No: 8 zum Nachweis von Pilzspezies der Gattung Aspergillus, wobei insbesondere A. fumigatus, A. flavus, A. versiculor, A. niger, A. nidulans und A. terreus nachgewiesen werden.

Die Erfindung betrifft ferner einen Kit zum Identifizieren von Pilzspezies, der an spezifische Nucleotidsequenzabschnitte der DNA der jeweiligen Pilzspezies hybridisierende DNA-Sonden enthält. Vorzugsweise enthält der Kit als DNA-Sonden eine oder mehrere der Nucleotidsequenzen SEQ ID-No: 3 - SEQ ID-No: 8 aus dem Sequenzprotokoll.

Ferner betrifft die Erfindung einen Kit zur Durchführung des gesamten oben genannten Verfahrens.

Hier ist von Vorteil, daß ein derartiger Kit entweder nur mit den DNA-Sonden oder aber zusätzlich mit weiter erforderlichen Lösungen bereitgestellt werden kann, so daß im Laboralltag sämtliche erforderlichen Stammlösungen etc. direkt aus diesem Kit genommen werden können. Dadurch vereinfacht sich der Nachweis, bzw. die Identifizierung der jeweiligen Pilzspezies erheblich, da die einzelnen Stammlösungen nicht mehr gesondert im Labor hergestellt werden müssen. Es kann aber auch möglich sein, in diesen Kit nur die Sonden und ggf. die Primer und Enzyme/Stammlösungen für die Polymerase-Kettenreaktion aufzunehmen, so daß im übrigen auf standardmäßig vorhandene Stammlösungen zurückgegriffen wird.

Ein die oben im einzelnen beschriebenen Verfahrensschritte in vorteilhafter Weise zusammenfassendes Verfahren zum Nachweisen von Pilzzellen in Vollblut umfaßt die Schritte:
- Isolation überwiegend intakter Pilzzellen aus dem Vollblut,
- Extrahieren von DNA aus den isolierten Pilzzellen,
- Amplifikation zumindest eines Segmentes der extrahierten Pilz-DNA, vorzugsweise zumindest eines Segmentes des PilzGens für die 18ssu-rRNA,
- Nachweis der Amplifikationsprodukte in Form einer Ja/Nein-Entscheidung, und
- Zuordnung der Amplifikationsprodukte zu einzelnen Pilz-Spezies durch Hybridisierung mit DNA-Sonden, die für bestimmte Pilzstämme und/oder -spezies spezifisch sind.

Ein großer Vorteil dieses zusammengefaßten Verfahrens liegt darin, daß nur ein Amplifikationsschritt erforderlich ist, durch den ein Segment der Pilz-DNA, vorzugsweise ein Segment des PilzGens für die 18ssu-rRNA derart hochverstärkt wird, daß diese Amplifikationsprodukte sowohl für die JA/Nein-Entscheidung als auch für die Bestimmung der Pilz-Spezies herangezogen werden können. Der Erfinder der vorliegenden Anmeldung hat nämlich erkannt, daß das Pilz-Gen für die 18ssu-rRNA einerseits zumindest abschnittweise durch zwei Primer flankiert werden kann, die für alle interessierenden Pilz-Spezies identisch sind, während das Amplikon andererseits für die verschiedenen Pilz-Spezies unterschiedlich ist, so daß es mit verschiedenen Sonden hybridisiert werden kann, die für die Pilz-Spezies kennzeichnend sind.

Mit anderen Worten, ein einziger Amplifikationsschritt reicht aus, um sowohl die Frage beantworten zu können, ob überhaupt eine Pilz-Infektion vorliegt, als auch die Frage, um welche Pilz-Spezies es sich handelt. Dieses Verfahren ist somit extrem einfach und leicht durchzuführen, Spezialkenntnisse sind nicht erforderlich, so daß auch angelerntes Personal eingesetzt werden kann.

Weitere Vorteile ergeben sich aus der nachstehenden Beschreibung.

Es versteht sich, daß die vorstehend genannten und die nachstehend noch zu erläuternden Merkmale nicht nur in den jeweils angegebenen Kombinationen, sondern auch in anderen Kombinationen oder in Alleinstellung verwendbar sind, ohne den Rahmen der vorliegenden Erfindung zu verlassen.

Beispiele für die Durchführung der einzelnen Verfahrensschritte sowie die Anwendung des Verfahrens im Rahmen eines klinischen Untersuchungsprogrammes sind in der folgenden Beschreibung angegeben.

Ein besonderer Vorteil des neuen Verfahrens liegt darin, daß es mit Vollblut durchgeführt wird, daß also z.B. keine Biopsie erforderlich ist oder noch herzustellendes Serum verwendet wird. Mit dem Vollblut wird zunächst eine Trennung der Pilzzellen von zellulärer DNA vorgenommen. Dies ist erforderlich, damit die ggf. nur in geringer Menge vorliegende Pilz-DNA nicht vor dem Hintergrund der in sehr viel höherer Konzentration vorhandenen zellulären DNA nachgewiesen werden muß. Dadurch wird also die Spezifität des Nachweisverfahrens erhöht. Zu diesem Zweck werden als erstes die roten Blutkörperchen durch osmotische Hämolyse lysiert und dann die weißen Blutkörperchen enzymatisch aufgeschlossen. Diese beiden Verfahrensschritte sind so ausgewählt, daß durch sie die Pilzzellen selbst noch nicht beschädigt werden und daß ggf. phagozytierte Pilzzellen unbeschädigt wieder freigesetzt werden, so daß auch sie für den weiteren Nachweis zur Verfügung stehen.

### Beispiel 1: Lysis roter Blutkörperchen durch osmotische Hämolyse.

Die Lysis der roten Blutkörperchen erfolgt mit Hilfe einer hypotonen Lösung. Dazu wird folgender Puffer mit folgenden Endkonzentrationen verwendet:

| | |
|---|---|
| Tris pH 7,6 | 10 mM |
| MgCl₂ | 5 mM |
| NaCl | 10 mM |

Die Lösung wird für 10 min bei Raumtemperatur inkubiert, und danach zentrifugiert.

Für diesen ersten Schritt ist eine Menge von 3 ml Vollblut ausreichend.

### Beispiel 2: Enzymatisches Aufschließen weißer Blutkörperchen

Die weißen Blutkörperchen, die ggf. Pilzzellen enthalten, werden vorsichtig aufgebrochen, indem die Zellen mit Proteinase K (200 µg/ml) der Firma Boehringer, Mannheim in folgendem Puffer mit den angegebenen Endkonzentrationen enzymatisch angedaut werden, in dem das Pellet aus Beispiel 1 aufgenommen wird:

| | |
|---|---|
| Tris pH 7,6 | 10 mM |
| EDTA pH 8,0 | 10 mM |
| NaCl | 50 mM |
| SDS | 0,2 % |
| Proteinase K | 200 µg/ml |

Dieser Puffer wird für zwei Stunden bei 65°C inkubiert.

### Beispiel 3: Trennung überwiegend intakter Pilzzellen vor allem von zellulärer DNA

Nachdem wie in den Beispielen 1 und 2 angegeben die Blutzellen aufgeschlossen wurden, so daß die zelluläre DNA freigegeben wurde, erfolgt ein Zentrifugationsschritt bei 5000 Umdrehungen/min, der zu einem erheblichen Verlust an zellulärer DNA führt, die bei dieser Zentrifugationsgeschwindigkeit nicht sedimentiert.

Im Sediment befinden sich jetzt vollständig die freien oder freigesetzten Pilzzellen, die für die weitere Verarbeitung in einem Puffer (Aqua bidest) aufgenommen werden.

### Beispiel 4: Aufschließen der Pilzzellen

Als nächstes werden die Pilzzellen alkalisch lysiert und enzymatisch behandelt, um die Pilz-DNA freizusetzen.

Dazu erfolgt zunächst eine Alkalilyse mit 200 ml 50 mM NaOH für 10 min bei 95°C.

Daraufhin erfolgt ein Neutralisationsschritt mit 1 M Tris-HCl pH 7,0.

Als nächstes werden 500 µl Zymolyase von Sigma (300 µg/ml) zugegeben und die Lösung für 60 min bei 37°C inkubiert, um die Pilzzellen enzymatisch aufzuschließen.

Daraufhin werden 500 µl Tris/EDTA und 50 µl 10%iger SDS Lösung zugegeben und das Gemisch bei 65°C für 20 min inkubiert, um das Protein zu denaturieren.

### Beispiel 5: Isolation der Pilz-DNA

In der nunmehr vorliegenden Lösung befinden sich Trümmer der Pilzzellen sowie freie Pilz-DNA, die nun isoliert werden muß.

Hierzu erfolgt zunächst eine Proteinpräzipitation mit 5 M Kaliumazetat, woraufhin der Überstand abgenommen und die DNA dann durch Zugabe von eiskaltem Isopropanol ausgefällt wird. Dieses Fällungsprodukt wird dann für die weiteren Verfahrensschritte verwendet.

Durch die in den Beispielen 1 - 5 angegebenen Verfahrensschritte ist es also möglich, aus Vollblut hoch-selektiv Pilz-DNA zu extrahieren, die nun als Präzipitat vorliegt und nur gering mit zellulärer DNA verunreinigt ist, wodurch der jetzt erfolgende Nachweis sehr sensitiv und hochspezifisch erfolgen kann.

### Beispiel 6: Amplifikation eines pilzspezifischen DNA-Segmentes

In diesem Verfahrensschritt soll zunächst nachgewiesen werden, ob in dem Präzipitat aus dem Verfahrensschritt in Beispiel 5 überhaupt Pilz-DNA vorhanden ist. Dabei wird ausgenutzt, daß die im Sequenzprotokoll unter SEQ ID-No: 1 und SEQ ID-No: 2 angegebenen DNA-Sequenzen spezifisch an Bindungsstellen auf dem Pilz-Gen für die 18ssu-rRNA vieler Pilzstämme und -spezies binden.

Der Erfinder der vorliegenden Anmeldung hat nämlich erkannt, daß dieses Pilz-Gen bei den verschiedenen Pilz-Stämmen und -Gattungen ein derartiges Sequenzsegment aufweist, das einerseits von zwei Bindungsregionen für Primer flankiert wird, die für alle Pilz-Stämme und -Gattungen identisch sind, daß aber andererseits die Sequenz dieses Segmentes für die verschiedenen Pilz-Stämme und -Gattungen so verschieden ist, daß sie gleichzeitig zum Nachweis der einzelnen Pilzspezies und -Gattungen verwendet werden kann.

Die DNA-Sequenz SEQ ID-No: 1 bindet dabei an den sense-Strang, während die DNA-Sequenz SEQ ID-No: 2 an den anti-sense-Strang bindet, wobei der Abstand zwischen den beiden Bindungsstellen ca. 500 Basenpaare beträgt. Diese beiden DNA-Sequenzen SEQ ID-No: 1 und SEQ ID-No: 2 eignen sich damit als Primer für eine Polymerase-Kettenreaktion (PCR), in der folglich Verstärkungsprodukte (Amplicon) mit einer Länge von ca. 500 Basenpaaren erzeugt werden.

In der nachfolgenden Tabelle 1 ist die Lage der Primer und die Länge des jeweiligen Amplicons angegeben.

**Tabelle 1: Von Pilz-Pathogenen erhaltene Amplicons**

| Pilzspezies | Bindungsstellen der Primer Vorwärts-Primer Rückwärts-Primer | | Länge des Amplicons |
|---|---|---|---|
| C. albicans | bp544-563 | bp1033-1014 | bp490 |
| C. glabrata | bp546-565 | bp1047-1028 | bp502 |
| C. krusei | bp535-554 | bp1016-997 | bp482 |
| C. tropicalis | bp544-563 | bp1030-1011 | bp487 |
| C. parapsilosis | bp544-563 | bp1033-1014 | bp490 |
| A. fumigatus | bp544-563 | bp1046-1027 | bp503 |
| A. niger | bp483-502 | bp986 - 967 | bp503 |
| A. flavus | bp483-502 | bp985 - 966 | bp502 |
| A. terreus | bp481-500 | bp984 - 965 | bp503 |
| A. nidulans | bp481-500 | bp984 - 965 | bp503 |

Durch diese beiden Primer SEQ ID-No: 1 und SEQ ID-No: 2 lassen sich also für alle relevanten Pilzspezies der Gattungen Candida und Aspergillus durch das PCR-Verfahren Amplicons von ca. 500 Basenpaaren in ausreichender Menge herstellen.

Die PCR-Bedingungen sind dabei die folgenden:

| | | |
|---|---|---|
| Puffer (50 µl): | | |
| 10 mM Tris pH 9,6 | | |
| 50 mM NaCl | | |
| 10 mM MgCl₂ | | |
| 0,2 mg/ml BSA | | |
| Polymerase | | |
| je 0,5 mM Nukleotide | | |
| je 100 pM Primer | | |
| Anfängliche Denaturierung: | 3 | min bei 94°C |
| Zyklus-Denaturierung: | 0,5 | min bei 94°C |
| Annealing: | 1 | min bei 62°C |
| Extension: | 2 | min bei 72°C |
| Terminale Extension: | 5 | min bei 72°C |
| Zykluszahl: | 34 | |

Die hohe Magnesiumkonzentration im Puffer sorgt für eine hohe Spezifität der Polymerase, die mit 72°C im Extensions-Schritt bei ihrem Temperaturoptimum arbeiten kann.

Mit diesen Primern konnten insgesamt 40 Stämme von Candida albicans, 10 Stämme von Candida tropicalis, 6 Stämme von Candida parapsilosis, 11 Stämme von Candida glabrata, 8 Stämme von Candida krusei, 8 Stämme von Aspergillus fumigatus, 6 Stämme von Aspergillus flavus, 5 Stämme von Aspergillus terreus, 7 Stämme von Aspergillus niger, 5 Stämme von Aspergillus nidulans und 3 Stämme von Aspergillus versiculor erfolgreich amplifiziert werden.

### Beispiel 7: Nachweis der Amplifikationsprodukte aus Beispiel 6

Im nächsten Schritt soll jetzt nachgewiesen werden, ob bei der PCR-Reaktion aus Beispiel 6 auch tatsächlich DNA-Segmente mit einer Länge von ca. 500 Basenpaaren hochverstärkt wurden. Diese Detektion der pilzspezifischen DNA-Segmente erfolgt durch Ethidiumbromid-Färbung der spezifischen Bande in einem 2%igen Agarose-Gel.

Ist hier die spezifische Bande zu erkennen, so kann von einer Pilzinfektion ausgegangen werden, da die Primer SEQ ID-No: 1 und SEQ ID-No: 2 an alle oben erwähnten Pilzstämme binden. Sollte also durch die Verfahrensschritte aus den Beispielen 1 - 5 Pilz-DNA extrahiert worden sein, so wird sie durch den PCR-Schritt des Beispiels 6 so weit hochverstärkt, daß sie hier durch Ethidiumbromid-Färbung nachgewiesen werden kann.

### Beispiel 8: Zuordnung der Amplifikationsprodukte aus Beispiel 6 zu einzelnen Pilzspezies

Für eine spezifische Therapie ist es jetzt noch erforderlich, die im Schritt 7 bereits nachgewiesene Pilzinfektion genauer zu spezifizieren. Hier zeigt sich jetzt ein weiterer Vorteil des PCR-Schrittes aus Beispiel 6. Dort wurde nämlich so viel pilzspezifisches DNA-Segment erzeugt, daß jetzt weitere Nachweisverfahren zur Bestimmung der Pilzspezies möglich sind.

Hierzu werden die im Sequenzprotokoll angegebenen Nucleotidsequenzen SEQ ID-No: 3 bis SEQ ID-No: 8 herangezogen, die als Spezies-spezifische Sonden dienen, die mit einem Sequenzabschnitt des in Beispiel 6 erzeugten DNA-Segmentes spezifisch hybridisieren.

Es wurde gefunden, daß die Sonde SEQ ID-No: 3 mit Candida albicans, SEQ ID-No: 4 mit Candida glabrata, SEQ ID-No: 5 mit Candida krusei, SEQ ID-No: 6 mit Candida tropicalis, SEQ ID-No: 7 mit Candida parapsilosis und SEQ ID-No: 8 mit Aspergillus fumigatus, A. flavus, A. versiculor, A. niger, A. nidulans und A. terreus hybridisieren. Die Nucleotidsequenz SEQ ID-No: 8 ist' also eine allgemeine Aspergillus-Sonde, während die Nucleotidsequenzen SEQ ID-No: 3 - SEQ ID-No: 5 Pilzspezies der Gattung Candida voneinander unterscheiden können.

Um die erfolgte Hybridisierung nachweisen zu können, werden die Sonden mit dem Transferase-Kit der Firma Boehringer, Mannheim mit Digoxigenin markiert, wobei der Nachweis nach dem Southern Blot-Verfahren mit der üblichen Farbreaktion erfolgt.

Mit Hilfe dieser Sonden erfolgt eine sequentielle Hybridisierung, die nach der Häufigkeit der einzelnen Pilzspezies gestaffelt ist, so daß zunächst mit SEQ ID-No: 3 (C. albicans), dann mit SEQ ID-No: 8 (Aspergillus), SEQ ID-No: 6 (C. tropicalis), SEQ ID-No: 7 (C. parapsilosis), SEQ ID-No: 4 (C. glabrata) und schließlich mit SEQ ID-No: 5 (C. krusei) hybridisiert wird.

Auf diese Weise ist es also möglich, an Hand der im Schrittbeispiel 6 erzeugten Amplifikationsprodukte durch sequentielles Hybridisieren die Pilzspezies zu identifizieren und anschließend eine gezielte Therapie einzuleiten.

### Beispiel 9: Nachweis ausgesäter Pilzzellen in Blut

Mit Hilfe der spezifischen Amplifikation und sequentiellen Hybridisierung gelang es, Pilzspezies mit einer Sensitivität von 1 - 3 Pilzzellen reproduzierbar nachzuweisen. Durch Aussaat von Pilzspezies in Blutproben konnte nachgewiesen werden, daß das obige Verfahren eine Sensitivität von einer sog. CFU (colony forming unit) /ml-Blut erreicht. Diese Nachweisgrenze liegt weit unterhalb derjenigen, die bei einer klinisch relevanten Pilzaussaat in Blut erwartet werden kann.

### Beispiel 10: Klinisches Untersuchungsprogramm

In einem groß angelegten klinischen Untersuchungsprogramm konnte eine hohe Spezifität des neuen Verfahrens bei der Analyse von 165 Blutproben von 65 gesunden Probanden gezeigt werden. Alle 165 Blutproben sind negativ geblieben.

94 immunsupprimierte Patenten mit unklarem Fieber wurden daraufhin auf die Präsenz einer Pilzinfektion untersucht. Von 69 Patienten, bei denen keine invasive Pilzinfektion vorlag, waren weit über 200 Blutproben (bis auf eine Ausnahme) ebenfalls negativ.

Dagegen konnten alle 25 Patienten mit invasiver Pilzinfektion bereits innerhalb der ersten Woche als positiv identifiziert werden. Bei allen Patienten gelang darüber hinaus die Zuordnung zu dem verursachenden Pilzerreger.

### SEQUENZPROTOKOLL

ALLGEMEINE ANGABEN:
   ANMELDER:
      NAME: Eberhard-Karls-Universität Tübingen, Universitätsklinikum
      STRASSE: Geissweg 3
      ORT: Tübingen
      LAND: Deutschland
      POSTLEITZAHL: 72076
      TELEFON: 07071-29-1
      TELEFAX: 07071-293966
   BEZEICHNUNG DER ERFINDUNG: Extraktion von Pilzzellen-DNA
   ANZAHL DER SEQUENZEN: 8
   COMPUTERLESBARE FASSUNG:
      DATENTRÄGER: (folgt)
      COMPUTER:
      BETRIEBSSYSTEM:
      SOFTWARE:
   DATEN DER JETZIGEN ANMELDUNG:
      ANWALTSAKTE: 5402P102
   ANGABEN ZU SEQ ID-NO:1:
      SEQUENZCHARAKTERISTIKA:
         LÄNGE: 20 Basenpaare
         ART: Nucleinsäure
         STRANGFORM: Einzelstrang
         TOPOLOGIE: linear
      HYPOTHETISCH: JA
         SEQUENZBESCHREIBUNG: SEQ ID-NO:1:
            ATTGGAGGGC AAGTCTGGTG 20
   ANGABEN ZU SEQ ID-NO:2:
      SEQUENZCHARAKTERISTIKA:
         LÄNGE: 20 Basenpaare
         ART: Nucleinsäure
         STRANGFORM: Einzelstrang
         TOPOLOGIE: linear
      HYPOTHETISCH: JA
      SEQUENZBESCHREIBUNG: SEQ ID-NO:2:
         CCGATCCCTA GTCGGCATAG 20
   ANGABEN ZU SEQ ID-NO:3:
      SEQUENZCHARAKTERISTIKA:
         LÄNGE: 30 Basenpaare
         ART: Nucleinsäure
         STRANGFORM: Einzelstrang
         TOPOLOGIE: linear
      HYPOTHETISCH: JA
      SEQUENZBESCHREIBUNG: SEQ ID-NO:3:
         TCTGGGTAGC CATTTATGGC GAACCAGGAC 30
   ANGABEN ZU SEQ ID-NO:4:
      SEQUENZCHARAKTERISTIKA:
         LÄNGE: 30 Basenpaare
         ART: Nucleinsäure
         STRANGFORM: Einzelstrang
         TOPOLOGIE: linear
      HYPOTHETISCH: JA
      SEQUENZBESCHREIBUNG: SEQ ID-NO:4:
         TTCTGGCTAA CCCCAAGTCC TTGTGGCTTG 30
   ANGABEN ZU SEQ ID-NO:5:
      SEQUENZCHARAKTERISTIKA:
         LÄNGE: 30 Basenpaare
         ART: Nucleinsäure
         STRANGFORM: Einzelstrang
         TOPOLOGIE: linear
      HYPOTHETISCH: JA
      SEQUENZBESCHREIBUNG: SEQ ID-NO:5:
         GTCTTTCCTT CTGGCTAGCC TCGGGCGAAC 30
   ANGABEN ZU SEQ ID-NO:6:
      SEQUENZCHARAKTERISTIRA:
         LÄNGE: 30 Basenpaare
         ART: Nucleinsäure
         STRANGFORM: Einzelstrang
         TOPOLOGIE: linear
      HYPOTHETISCH: JA
      SEQUENZBESCHREIBUNG: SEQ ID-NO:6:
         GTTGGCCGGT CCATCTTTCT GATGCGTACT 30
   ANGABEN ZU SEQ ID-NO:7:
      SEQUENZCHARAKTERISTIKA:
         LÄNGE: 30 Basenpaare
         ART: Nucleinsäure
         STRANGFORM: Einzelstrang
         TOPOLOGIE: linear
      HYPOTHETISCH: JA
      SEQUENZBESCHREIBUNG: SEQ ID-NO:7:
         TTTCCTTCTG GCTAGCCTTT TTGGCGAACC 30
   ANGABEN ZU SEQ ID-NO:8:
      SEQUENZCHARAKTERISTIKA:
         LÄNGE: 30 Basenpaare
         ART: Nucleinsäure
         STRANGFORM: Einzelstrang
         TOPOLOGIE: linear
      HYPOTHETISCH: JA
      SEQUENZBESCHREIHUNG: SEQ ID-NO:8:
         CATGGCCTTC ACTGGCTGTG GGGGGAACCA 30

## Patentansprüche

1. Nukleötidsequenz SEQ ID-No: 1 aus dem beiliegenden Sequenzprotokoll.

2. Nukleotidsequenz SEQ ID-No: 2 aus dem beiliegenden Sequenzprotokoll.

3. Verwendung der Nukleotidsequenzen aus den Ansprüchen 1 und 2 als Primer zur Amplifikation eines Abschnitts aus dem Pilzgen für 18ssu-rRNA von einer Vielzahl von Pilzspezies in einer Polymerase-Kettenreaktion.

4. Verfahren zum Nachweis von Pilz-DNA in klinischem Material unter Extrahieren von Pilz-DNA aus Vollblut, mit den Schritten:
a) Isolation überwiegend intakter Pilzzellen aus dem Vollblut durch
a1) Aufschließen der im Vollblut befindlichen Blutzellen;
a2) Isolation überwiegend intakter Pilzzellen von zellulärer DNA;
b) Extrahieren von DNA aus den isolierten Pilzzellen durch
b1) Aufschließen der isolierten Pilzzellen, und
b2) Isolation der Pilz-DNA;
c) Amplifikation zumindest eines Segments der nachzuweisenden Pilz-DNA durch Polymerase-Kettenreaktion unter Verwendung der Nukleotidsequenzen SEQ ID-No: 1 und SEQ ID-No: 2 aus dem beiliegenden Sequenzprotokoll als Primer, und
d) gegebenenfalls Nachweis der Ainplifikationsprodukte.

5. Verfahren nach Anspruch 4, **dadurch gekennzeichnet, daß** der Schritt a) die weiteren Schritte umfaßt:
a1.1) Lysis der roten Blutkörperchen durch osmotische Hämolyse;
a1.2) enzymatisches Aufschließen der weißen Blutkörperchen; und
a2.1) Zentrifugation des so behandelten Vollblutes und Verwendung des Pellet in den nächsten Verfahrensschritten.

6. Verfahren nach Anspruch 5, **dadurch gekennzeichnet, daß** im Schritt a1.1) die Lysis der roten Blutkörperchen mit Hilfe einer hypotonen Lösung und einem Detergenz erfolgt, vorzugsweise mit einer Endkonzentration von 10 mM Tris pH 7, 6, 5 mM MgCl₂ und 10 mM NaCl.

7. Verfahren nach Anspruch 5 oder 6, **dadurch gekennzeichnet, daß** im Schritt a1.2) das enzymatische Aufschließen der weißen Blutkörperchen in einer Lösung mit einer Endkonzentration von 200 µg/ml Proteinase K, 10 mM Tris pH 7, 6, 10 mM EDTA pH 8, 0, 50 mM NaCl und 0,2 % SDS erfolgt.

8. Verfahren nach Anspruch 7, **dadurch gekennzeichnet, daß** die Lösung für 100 - 140 min, vorzugsweise 120 min bei 60-70°C, vorzugsweise bei 65°C inkubiert wird.

9. Verfahren nach einem der Ansprüche 4 bis 8 **dadurch gekennzeichnet, daß** der Schritt b1) den weiteren Schritt umfaßt:
b1.1) Alkalisches Lysieren und enzymatische Behandlung der Pilzzellen.

10. Verfahren nach Anspruch 9, **dadurch gekennzeichnet, daß** der Schritt b1.1) folgende Schritte umfaßt:
- Inkubieren der im Pellet aus Schritt a2.1) befindlichen Pilzzellen bei 90-95°C, vorzugsweise 95°C für 5-15 min, vorzugsweise 10 min, in einer Lösung mit 50 mM NaOH, und
- Neutralisation mit 1 M Tris-HCl pH 7,0,
- enzymatische Behandlung mit Zymolyase für 50-70 min, vorzugsweise 60 min bei 30-40°C, vorzugsweise 37°C,
- Proteindenaturierung durch Inkubation mit Tris/EDTA bei 60-70 C, vorzugsweise 65°C für 10-30 min, vorzugsweise 20 min.

11. Verfahren nach Anspruch 9 oder 10, **dadurch gekennzeichnet, daß** der Schritt b2) folgende Schritte umfaßt:
- Proteinpräzipitation mit 5 M Kaliumazetat, und
- DNA-Präzipitation des Überstandes in eiskaltem Isopropanol.

12. Verfahren nach einem der Ansprüche 4 bis 11, **dadurch gekennzeichnet, daß** im Schritt c) die Polymerase-Kettenreaktion mit folgendem Zyklus durchgeführt wird:
c1) Denaturieren für 0,3-1 min, vorzugsweise für 0,5 min bei 90-95°C, vorzugsweise bei 94°C,
c2) Hybridisieren für 0,5-1,5 min, vorzugsweise für 1,0 min bei 58-64°C, vorzugsweise bei 62°C, und
c3) Extension für 1,5-2,5 min, vorzugsweise für 2,0 min bei 68-75°C, vorzugsweise bei 72°C.

13. Verfahren nach Anspruch 12, **dadurch gekennzeichnet, daß** vor Beginn der Zyklusschritte ein Denaturierungsschritt von 5-9 min, vorzugsweise von 3 min bei 90-96°C, vorzugsweise bei 94°C erfolgt.

14. Verfahren nach einem der Ansprüche 4 bis 11 oder 12 oder 13, **dadurch gekennzeichnet, daß** im Schritt d) die Amplifikationsprodukte gelelektrophoretisch, vorzugsweise auf einem Agarosegel, nachgewiesen und dazu vorzugsweise mit Ethidiumbromid eingefärbt werden.

15. Kit zum Nachweisen von Pilz-DNA in einer Untersuchungslösung, **dadurch gekennzeichnet, daß** er als Primer die Nukleotidsequenzen aus Anspruch 1 und 2 enthält.

16. Nukleotidsequenz SEQ ID-No: 3 aus dem beiliegenden Sequenzprotokoll.

17. Nukleotidsequenz SEQ ID-No: 4 aus dem beiliegenden Sequenzprotokoll.

18. Nukleotidsequenz SEQ ID-No: 5 aus dem beiliegenden Sequenzprotokoll.

19. Nukleotidsequenz SEQ ID-No: 6 aus dem beliegenden Sequenzprotokoll.

20. Nukleotidsequenz SEQ ID-No: 7 aus dem beiliegenden Sequenzprotokoll.

21. Nukleotidsequenz SEQ ID-No: 8 aus dem beiliegenden Sequenzprotokoll.

22. Verwendung einer oder mehrerer der Nukleotidsequenzen aus den Ansprüchen 16 bis 21 als DNA-Sonde zum Identifizieren von Pilzspezies.

23. Verwendung der Nukleotidsequenz aus Anspruch 16 zum Nachweis der Pilzspezies Candida albicans.

24. Verwendung der Nukleotidsequenz aus Anspruch 17 zum Nachweis der Pilzspezies Candida glabrata.

25. Verwendung der Nukleotidsequenz aus Anspruch 18 zum Nachweis der Pilzspezies Candida krusei.

26. Verwendung der Nukleotidsequenz aus Anspruch 19 zum Nachweis der Pilzspezies Candida tropicalis.

27. Verwendung der Nukleotidsequenz aus Anspruch 20 zum Nachweis der Pilzspezies Candida parapsilosis.

28. Verwendung der Nukleotidsequenz aus Anspruch 21 zum Nachweis von Pilzspezies der Gattung Aspergillus.

29. Verfahren zum Identifizieren von Pilzspezies, vorzugsweise durchzuführen im Zusammenhang mit dem Verfahren nach einem der Ansprüche 4 bis 11 oder 12 bis 14, mit dem zusätzlichen Schritt:
e) Bestimmen von für die Pilzspezies kennzeichnenden Nukleotidsequenzabschnitten der in der Untersuchungslösung enthaltenen DNA,
wobei in Schritt e) die Pilz-DNA oder Segmente der Pilz-DNA, die ggf. zuvor gemäß Schritt c) amplifiziert wurden, mit DNA-Sonden hybridisiert werden, die für bestimmte Pilzstämme und/oder -spezies spezifisch sind, und wobei der Test der erfolgten Hybridisierung dann zur Identifizierung der Pilzspezies führt, **dadurch gekennzeichnet, daß** als DNA-Sonden eine oder mehrere der Nukleotidsequenzen SEQ ID-No: 3 bis SEQ ID-No: 8 aus dem beiliegenden Sequenzprotokoll verwendet werden.

30. Verfahren nach Anspruch 29, **dadurch gekennzeichnet, daß** zum Testen der erfolgten Hybridisierung die Sonden mit Digoxigenin markiert und die Hybride z.B. nach der Southern Blot-Methode nachgewiesen werden.

31. Verfahren nach Anspruch 29 oder 30, **dadurch gekennzeichnet, daß** die DNA-Sonden sequentiell nacheinander, vorzugsweise in der Reihenfolge SEQ ID-No: 3, SEQ ID-No: 8, SEQ ID-No: 6, SEQ ID-No: 7, SEQ ID-No: 4 und SEQ ID-No: 5 eingesetzt werden und jeweils die Hybridisierung getestet wird.

32. Kit zum Identifizieren von Pilzspezies, **dadurch gekennzeichnet, daß** er als DNA-Sonden eine oder mehrere der Nukleotidsequenzen aus den Ansprüchen 16 bis 21 enthält.

33. Verfahren zum Nachweisen von Pilzzellen in Vollblut, mit den Schritten:
- Isolation überwiegend intakter Pilzzellen aus dem Vollblut,
- Extrahieren von DNA aus den isolierten Pilzzellen,
- Amplifikation zumindest eines Segmentes der extrahierten Pilz-DNA, und zwar eines Segmentes des Pilzgens für die 18ssu-rRNA mit den Nukleotidsequenzen SEQ ID-No: 1 und 2 aus dem beiliegenden Sequenzprotokoll,
- Nachweis der Amplifikationsprodukte in Form einer ja/nein-Entscheidung, und
- Zuordnung der Amplifikationsprodukte zu einzelnen Pilzspezies durch Hybridisierung mit einer oder mehreren der Nukleotidsequenzen SEQ ID-No: 3 bis 8 als DNA-Sonden.

## Claims

1. Nucleotide sequence SEQ ID-No: 1 of the appended sequence listing.

2. Nucleotide sequence SEQ ID-No: 2 of the appended sequence listing.

3. Use of the nucleotide sequences of Claims 1 and 2 as primers for the amplification of a segment from the fungal gene for 18ssu-rRNA from a multiplicity of fungal species in a polymerase chain reaction.

4. Method of detecting fungal DNA in clinical material involving the extraction of fungal DNA from whole blood, comprising the following steps:
a) isolation of predominantly intact fungal cells from the whole blood by
a1) disrupting the haematocytes present in the whole blood;
a2) isolation of predominantly intact fungal cells from cellular DNA;
b) extraction of DNA from the isolated fungal cells by
b1) disrupting the isolated fungal cells, and
b2) isolation of the fungal DNA;
c) amplification by polymerase chain reaction of at least one segment of the fungal DNA to be detected using the nucleotide sequences SEQ ID-No: 1 and SEQ ID-No: 2 from the appended sequence listing as primers, and
d) if appropriate, detection of the amplificates.

5. Method according to Claim 4, **characterized in that** step a) comprises the following further steps:
a1.1) lysis of the erythrocytes by osmotic haemolysis;
a1.2) enzymatic disruption of the leucocytes; and
a2.1) centrifugation of the whole blood treated thus, and use of the pellet in the following methodological steps.

6. Method according to Claim 5, **characterized in that**, in step a1.1), the lysis of the erythrocytes with the aid of a hypotonic solution and a detergent, preferably with a final concentration of 10 mM Tris pH 7.6, 5 mM Much and 10 mM NaCl.

7. Method according to Claim 5 or 6, **characterized in that**, in step a1.2), the enzymatic disruption of the leucocytes is performed in a solution with a final concentration of 200 µg/ml Proteinase K, 10 mM Tris pH 7.6, 10 mM EDTA pH 8.0, 50 mM NaCl and 0.2% SDS.

8. Method according to Claim 7, **characterized in that** the solution is incubated for 100-140 min, preferably 120 min, at 60-70°C, preferably at 65°C.

9. Method according to one of Claims 4 to 8, **characterized in that** step b1) comprises the further step:
b1.1) subjecting the fungal cells to alkaline lysis and enzymatic treatment.

10. Method according to Claim 9, **characterized in that** step b1.1) comprises the following steps:
- incubation of the fungal cells present in the pellet of step a2.1) at 90-98°C, preferably 95°C, for 5-15 min, preferably 10 min, in a solution with 50 mM NaOH,
- neutralization with 1 M Tris-HCl pH 7.0,
- enzymatic treatment with Zymolyase for 50-70 min, preferably 60 min, at 30-40°C, preferably 37°C,
- protein denaturation by incubation with Tris/EDTA at 60-70°C, preferably 65°C, for 10-30 min, preferably 20 min.

11. Method according to Claim 9 or 10, **characterized in that** step b2) comprises the following steps:
- protein precipitation with 5 M potassium acetate, and
- DNA precipitation of the supernatant in ice-cold isopropanol.

12. Method according to one of Claims 4 to 11, **characterized in that**, in step c), the polymerase chain reaction is carried out with the following cycle:
c1) denaturation for 0.3-1 min, preferably for 0.5 min, at 90-95°C, preferably at 94°C;
c2) hybridization for 0.5-1.5 min, preferably for 1.0 min at 58-64°C, preferably at 62°C, and
c3) extension for 1.5-2.5 min, preferably for 2.0 min, at 68-75°C, preferably at 72°C.

13. Method according to Claim 12, **characterized in that** a denaturation step of 5-9 min, preferably 3 min, at 90-96°C, preferably at 94°C, is carried out before the steps of the cycle are started.

14. Method according to one of Claims 4 to 11 or 12 or 13, **characterized in that**, in step d), the amplificates are detected by gel electrophoresis, preferably on an agarose gel, for which purpose they are preferably stained with ethidium bromide.

15. Kit for detecting fungal DNA in a test solution, **characterized in that** it contains the nucleotide sequences of Claim 1 and 2 as primers.

16. Nucleotide sequence SEQ ID-No: 3 of the appended sequence listing.

17. Nucleotide sequence SEQ ID-No: 4 of the appended sequence listing.

18. Nucleotide sequence SEQ ID-No: 5 of the appended sequence listing.

19. Nucleotide sequence SEQ ID-No: 6 of the appended sequence listing.

20. Nucleotide sequence SEQ ID-No: 7 of the appended sequence listing.

21. Nucleotide sequence SEQ ID-No: 8 of the appended sequence listing.

22. Use of one or more of the nucleotide sequences of Claims 16 to 21 as DNA probe for identifying fungal species.

23. Use of the nucleotide sequence of Claim 16 for detecting the fungal species Candida albicans.

24. Use of the nucleotide sequence of Claim 17 for detecting the fungal species Candida glabrata.

25. Use of the nucleotide sequence of Claim 18 for detecting the fungal species Candida krusei.

26. Use of the nucleotide sequence of Claim 19 for detecting the fungal species Candida tropicalis.

27. Use of the nucleotide sequence of Claim 20 for detecting the fungal species Candida parapsilosis.

28. Use of the nucleotide sequence of Claim 21 for detecting fungal species of the genus Aspergillus.

29. Method of identifying fungal species, preferably to be carried out in connection with the method according to one of Claims 4 to 11 or 12 to 14, with the additional step:
e) determination of nucleotide sequence segments of the DNA present in the test solution, which segments are characteristic of the fungal species,
where, in step e), the fungal DNA or segments of the fungal DNA which, if appropriate, have previously been amplified in accordance with step c), are hybridized with DNA probes which are specific for certain fungal strains and/or fungal species, and where the assay whether hybridization has taken place then leads to the identification of the fungal species, **characterized in that** the DNA probes employed are one or more of the nucleotide sequences SEQ ID-No: 3 to SEQ ID-No: 8 of the appended sequence listing.

30. Method according to Claim 29, **characterized in that**, to assay whether hybridization has taken place, the probes are labelled with digoxigenin and the hybrids are detected for example by means of the Southern blot method.

31. Method according to Claim 29 or 30, **characterized in that** the DNA probes are employed sequentially one after the other, preferably in the sequence SEQ ID-No: 3, SEQ ID-No: 8, SEQ ID-No: 6, SEQ ID-No: 7, SEQ ID-No: 4 and SEQ ID-No: 5, and hybridization is assayed in each case.

32. Kit of identifying fungal species, **characterized in that** it contains one or more of the nucleotide sequences of Claims 16 to 21 as DNA probes.

33. Method of detecting fungal cells in whole blood, comprising the steps:
- isolation of predominantly intact fungal cells from the whole blood,
- extraction of DNA from the isolated fungal cells,
- amplification of at least one segment of the extracted fungal DNA, which is a segment of the fungal gene for the 18ssu-rRNA, with the nucleotide sequences SEQ ID-No: 1 and 2 of the appended sequence listing,
- detection of the amplificates in the form of a yes/no decision, and
- assignation of the amplificates to individual fungal species by hybridization with one or more of the nucleotide sequences SEQ ID-No: 3 to 8 as DNA probes.

## Revendications

1. Séquence nucléotidique SEQ ID-No : 1 du listage de séquences joint.

2. Séquence nucléotidique SEQ ID-No : 2 du listage de séquences joint.

3. Utilisation des séquences nucléotidiques des revendications 1 et 2 comme amorces pour l'amplification d'un fragment provenant du gène fongique d'ARNr 18ssu d'une multiplicité d'espèces fongiques dans une réaction en chaîne par polymérase.

4. Procédé pour la mise en évidence d'ADN fongique dans un matériel clinique avec extraction d'ADN fongique du sang total, comportant les étapes :
a) isolement de cellules fongiques pour la plupart intactes à partir du sang total par
a1) désagrégation des cellules sanguines qui se trouvent dans le sang total ;
a2) isolement de cellules fongiques pour la plupart intactes de l'ADN cellulaire ;
b) extraction de l'ADN à partir des cellules fongiques isolées par
b1) désagrégation des cellules fongiques isolées, et
b2) isolement de l'ADN fongique ;
c) amplification d'au moins un segment de l'ADN fongique qui doit être mis en évidence par réaction en chaîne par polymérase en utilisant les séquences nucléotidiques SEQ ID-No : 1 et SEQ ID-No : 2 du listage de séquences joint comme amorces, et
d) éventuellement mise en évidence des produits d'amplification.

5. Procédé selon la revendication 4 **caractérisé en ce que** l'étape a) comprend les autres étapes :
a1.1) lyse des globules rouges par hémolyse osmotique ;
a1.2) désagrégation enzymatique des globules blancs ; et
a2.1) centrifugation du sang total ainsi traité et utilisation du culot dans les étapes du procédé suivantes.

6. Procédé selon la revendication 5 **caractérisé en ce que**, dans l'étape a1.1), la lyse des globules rouges a lieu à l'aide d'une solution hypotonique et d'un détergent, de préférence avec une concentration finale de 10 mM de Tris pH 7,6, 5 mM de MgCl₂ et 10 mM de NaCl.

7. Procédé selon la revendication 5 ou 6 **caractérisé en ce que**, dans l'étape a1.2), la désagrégation enzymatique des globules blancs a lieu dans une solution avec une concentration finale de 200 µg/ml de protéinase K, 10 mM de Tris pH 7,6, 10 mM d'EDTA pH 8,0, 50 mM de NaCl et 0,2 % de SDS.

8. Procédé selon la revendication 7 **caractérisé en ce que** la solution est incubée pendant 100-140 min, de préférence 120 min à 60-70°C, de préférence à 65°C.

9. Procédé selon l'une des revendications 4 à 8 **caractérisé en ce que** l'étape b1) comprend l'autre étape :
b1.1) lyse alcaline et traitement enzymatique des cellules fongiques.

10. Procédé selon la revendication 9 **caractérisé en ce que** l'étape b1.1) comprend les étapes suivantes :
- incubation des cellules fongiques qui se trouvent dans le culot provenant de l'étape a2.1) à 90-95°C, de préférence 95°C pendant 5-15 min, de préférence 10 min, dans une solution avec NaOH 50 mM, et
- neutralisation avec Tris-HCl pH 7,0 1 M,
- traitement enzymatique avec la zymolase pendant 50-70 min, de préférence 60 min à 30-40°C, de préférence 37°C,
- dénaturation des protéines par incubation avec Tris/EDTA à 60-70°C, de préférence 65°C pendant 10-30 min, de préférence 20 min.

11. Procédé selon la revendication 9 ou 10 **caractérisé en ce que** l'étape b2) comprend les étapes suivantes :
- précipitation des protéines avec de l'acétate de potassium 5 M, et
- précipitation de l'ADN du surnageant dans de l'isopropanol glacé.

12. Procédé selon l'une des revendications 4 à 11 **caractérisé en ce que**, dans l'étape c), la réaction en chaîne par polymérase est conduite avec le cycle suivant :
c1) dénaturation pendant 0,3-1 min, de préférence pendant 0,5 min à 90-95°C, de préférence à 94°C,
c2) hybridation pendant 0,5-1,5 min, de préférence pendant 1,0 min à 58-64°C, de préférence à 62°C, et
c3) extension pendant 1,5-2,5 min, de préférence pendant 2,0 min à 68-75°C, de préférence à 72°C.

13. Procédé selon la revendication 12 **caractérisé en ce que**, avant le début des étapes du cycle a lieu une étape de dénaturation de 5-9 min, de préférence de 3 min à 90-96°C, de préférence à 94°C.

14. Procédé selon l'une des revendications 4 à 11 ou 12 ou 13 **caractérisé en ce que**, dans l'étape d), les produits d'amplification sont mis en évidence par électrophorèse en gel, de préférence sur un gel d'agarose, et pour ce faire sont de préférence colorés avec le bromure d'éthidium.

15. Kit pour la mise en évidence d'ADN fongique dans une solution en étude, **caractérisé en ce qu'**il contient comme amorces les séquences nucléotidiques des revendications 1 et 2.

16. Séquence nucléotidique SEQ ID-No : 3 du listage de séquences joint.

17. Séquence nucléotidique SEQ ID-No : 4 du listage de séquences joint.

18. Séquence nucléotidique SEQ ID-No : 5 du listage de séquences joint.

19. Séquence nucléotidique SEQ ID-No : 6 du listage de séquences joint.

20. Séquence nucléotidique SEQ ID-No : 7 du listage de séquences joint.

21. Séquence nucléotidique SEQ ID-No : 8 du listage de séquences joint.

22. Utilisation d'une ou plusieurs des séquences nucléotidiques des revendications 16 à 21 comme sonde ADN pour l'identification d'espèces fongiques.

23. Utilisation de la séquence nucléotidique de la revendication 16 pour la mise en évidence de l'espèce fongique Candida albicans.

24. Utilisation de la séquence nucléotidique de la revendication 17 pour la mise en évidence de l'espèce fongique Candida glabrata.

25. Utilisation de la séquence nucléotidique de la revendication 18 pour la mise en évidence de l'espèce fongique Candida krusei.

26. Utilisation de la séquence nucléotidique de la revendication 19 pour la mise en évidence de l'espèce fongique Candida tropicalis.

27. Utilisation de la séquence nucléotidique de la revendication 20 pour la mise en évidence de l'espèce fongique Candida parapsilosis.

28. Utilisation de la séquence nucléotidique de la revendication 21 pour la mise en évidence d'espèces fongiques du genre Aspergillus.

29. Procédé pour l'identification d'espèces fongiques, de préférence destiné à être mis en oeuvre en liaison avec le procédé selon l'une des revendications 4 à 11 ou 12 à 14, comportant l'étape supplémentaire :
e) détermination de fragments de séquences nucléotidiques caractéristiques des espèces fongiques de l'ADN contenu dans la solution en étude,
où, dans l'étape e), l'ADN fongique ou des segments de l'ADN fongique, qui ont éventuellement été amplifiés précédemment selon l'étape c), sont hybridés avec des sondes ADN qui sont spécifiques de souches fongiques et/ou d'espèces fongiques déterminées, et où le test de l'hybridation effective conduit ensuite à l'identification des espèces fongiques, **caractérisé en ce qu'**une ou plusieurs des séquences nucléotidiques SEQ ID-No : 3 à SEQ ID-No : 8 du listage de séquences joint sont utilisées comme sondes ADN.

30. Procédé selon la revendication 29 **caractérisé en ce que**, pour tester l'hybridation effective, les sondes sont marquées avec la digoxigénine et les hybrides sont mis en évidence par exemple selon la méthode de Southem blot.

31. Procédé selon la revendication 29 ou 30 **caractérisé en ce que** les sondes ADN sont utilisées successivement les unes après les autres, de préférence dans l'ordre SEQ ID-No : 3, SEQ ID-No : 8, SEQ ID-No : 6, SEQ ID-No : 7, SEQ ID-No : 4 et SEQ ID-No : 5 et l'hybridation est testée à chaque fois.

32. Kit pour l'identification d'espèces fongiques **caractérisé en ce qu'**il contient comme sondes ADN une ou plusieurs des séquences nucléotidiques des revendications 16 à 21.

33. Procédé pour la mise en évidence de cellules fongiques dans du sang total comportant les étapes :
- isolement de cellules fongiques pour la plupart intactes à partir du sang total,
- extraction d'ADN à partir des cellules fongiques isolées,
- amplification d'au moins un segment de l'ADN fongique extrait, et ce d'un segment du gène fongique de l'ARNr 18ssu avec les séquences nucléotidiques SEQ ID-No : 1 et 2 du listage de séquences joint,
- mise en évidence des produits d'amplification sous forme d'une décision oui/non et
- attribution des produits d'amplification à des espèces fongiques individuelles par hybridation avec une ou plusieurs des séquences nucléotidiques SEQ ID-No : 3 à 8 comme sondes ADN.
